# EUROPÄISCHE PATENTSCHRIFT

(11) **EP 0 860 415 B1**
(45) Veröffentlichungstag und Bekanntmachung des Hinweises auf die Patenterteilung: **04.12.2002**
(21) Anmeldenummer: 98102955.6
(22) Anmeldetag: 20.02.1998
(51) Int. Cl.: C07C 29/56, C07C 33/02

(54) **Verfahren zur Isomerisierung von Allylalkoholen**
Process for the isomerisation of allyl alcohols
Procédé pour l'isomérisation d'alcohols allyliques

(30) Priorität: 25.02.1997 DE 19707385; 01.09.1997 DE 19738083
(43) Veröffentlichungstag der Anmeldung: 26.08.1998
(73) Patentinhaber: BASF AKTIENGESELLSCHAFT, 67056 Ludwigshafen (DE)
(72) Erfinder: Kiefer, Matthias, Dr., 69226 Nussloch (DE); Siegel, Wolfgang, Dr., 67117 Limburgerhof (DE); Therre, Jörg, Dr., 67550 Worms (DE); Pahl, Melanie, 68307 Mannheim (DE); Aquila, Werner, Dr., 68309 Mannheim (DE); Schäfer-Lüderssen, Ulrich, Dr., 67063 Ludwigshafen (DE); Rheude, Udo, Dr., 67166 Otterstadt (DE)

(56) Entgegenhaltungen:
- DE-A- 1 925 197
- DATABASE WPI Section Ch, Week 7926 Derwent Publications Ltd., London, GB; Class B05, AN 79-47786B XP002061761 & JP 54 061 110 A (KURARAY CO LTD)

## Beschreibung

Die vorliegende Erfindung betrifft ein Verfahren zur Isomerisierung von Edukt-Allylalkoholen zu Produkt-Allylalkoholen in wäßriger Lösung in Gegenwart von Protonensäuren.

Allylalkohole sind wichtige Zwischenprodukte in der industriellen organischen Chemie. Tertiäre Allylalkohole insbesondere dienen zum Beispiel als Zwischenstufen bei der Herstellung von Riechstoffen oder auch als Additive in Seifen oder Detergentien.

Es ist bekannt, daß Allylalkohole unter Säurekatalyse isomerisieren. Diese Isomerisierung entspricht einer 1,3-Wanderung der Hydroxygruppe und einer entsprechenden Verschiebung der Doppelbindung, wie in der folgenden Gleichung mit den allgemeinen Formeln I und II dargestellt:

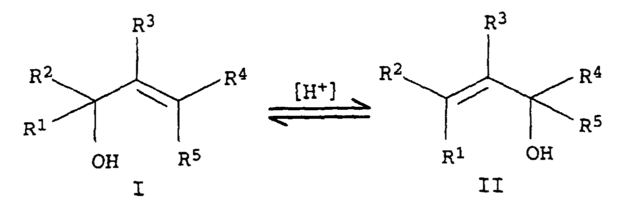

Diese Wanderung einer Doppelbindung und eines Substituenten ist von Allylverbindungen bekannt und wird im allgemeinen als Allyl-Umlagerung bezeichnet. Allyl-Umlagerungen von Allylalkoholen sind Gleichgewichtsreaktionen.

Eine allgemeine Übersicht über die durch Protonensäuren katalysierte Isomerisierung von Allylalkoholen gibt z. B. Houben-Weyl:"Methoden der organischen Chemie", Band VI, 1b, Seite 528ff, Stuttgart 1984. Dort wird unter anderem beschrieben, daß sich besonders einfach solche Isomerisierungen durchführen lassen, bei denen aus einem tertiären Allylalkohol mit endständiger C=C-Doppelbindung der entsprechende primäre Alkohol mit innenständiger C=C-Doppelbindung entsteht.

Dementsprechend wurde in der Vergangenheit industriell vor allem die Isomerisierung von Dimethylvinylcarbinol (DMVC, 2-Methylbut-3-en-2-ol), einem tertiären Allylalkohol, zu seinem Isomeren Prenol (3-Methyl-but-2-enol), einem primären Allylalkohol, genutzt. Auf Basis der früher weit verbreiteten technischen Carbid-Acetylenchemie waren Dialkyl-Alkenylcarbinole wie zum Beispiel DMVC durch baseninduzierte Kondensation von 1-Alkinen wie zum Beispiel Acetylen mit Ketonen wie zum Beispiel Aceton und nachfolgende Hydrierung der Dreifach- zur Doppelbindung leicht zu gänglich. Heutzutage ist Acetylen ein vergleichsweise seltener und teurer Rohstoff. Auf der heute üblichen petrochemischen Rohstoffbasis sind dagegen Olefine wie Dialkylalkene, zum Beispiel Isobuten, leicht zugänglich, die durch Kondensation mit Aldehyden, wie zum Beispiel Formaldehyd, und nachfolgende Isomerisierung der Doppelbindung leicht zu Prenol oder Prenol-Derivaten wie zum Beispiel mit organischen Resten substituierten Prenolen verarbeitet werden können. Technisch besteht daher heute vor allem Bedarf an einem Verfahren, mit dem aus primären Allylalkoholen wie Prenol tertiäre Allylalkohole wie DMVC hergestellt werden können.

SU-A 181 090 beschreibt ein Verfahren zur Isomerisierung von Prenol zu DMVC, bei dem Mineralsäure, insbesondere Schwefelsäure, in einer Konzentration unter 1 Gew.-% (entsprechend einem pH-Wert oberhalb von 0,7), vorzugsweise 0,3 bis 0,5 Gew.-% als Katalysator eingesetzt wird. Dieser bevorzugte Konzentrationsbereich entspricht einem pH-Bereich von 1,0 bis 1,2. Diese Schrift lehrt aber auch, daß bei der Durchführung des offenbarten Verfahrens in hohen Mengen Nebenprodukte anfallen, die regelmäßig aus dem Reaktor entfernt werden müssen.

A.I. Lebedeva und L.L. Shchukovskaya, J. Gen. Chem. USSR, 21 (1951) 1235 - 1241 untersuchten die Abhängigkeit der Isomerisierung von DMVC vom pH-Wert des Reaktionsmediums und von der angewendeten Reaktionstemperatur bei Reaktionsdauern von 30 Stunden. Sie stellten fest, daß erst bei einem pH-Wert von 1,29 oder kleiner eine Isomerisierung des eingesetzten DMVC bei Raumtemperatur festzustellen war. Bei einem pH-Wert von 1,32 oder darüber wurde weder bei Raumtemperatur noch in einem siedenden Wasserbad irgendeine Isomerisierung des DMVC beobachtet.

I. N. Nazarov, I. N. Azerbaev und V. N. Rakcheeva lehren in Bull. Acad. Sci. USSR, Chem. Ser. 1946, 419 - 426, daß die Isomerisierung von Dialkylvinylcarbinolen, also den tertiären Allylalkoholen, in die entsprechenden primären Allylalkohole in einem Temperaturbereich von 60 bis 100 °C unter dem Einfluß von 0,1 %iger Schwefelsäure, also bei einem pH-Wert von etwa 1,7, "ziemlich glatt" verläuft, während bei Raumtemperatur die Isomerisierung unter Einfluß von 1 - 5 %iger Schwefelsäure, also pH-Werten von etwa 0,7 und darunter durchgeführt wird. Diese Veröffentlichung lehrt weiterhin, daß bei einer Schwefelsäurekonzentration von 0,01 %, also einem pH-Wert von 2,7, der Isomerisationsprozeß selbst bei einer Temperatur von 100°C zu langsam verläuft.

Die bekannten Beispiele für die Isomerisierung von Allylalkoholen wurden daher sämtlich mit hohen Säurekonzentrationen, also niedrigen pH-Werten von höchstens 1,5 oder sogar deutlich unterhalb von 1 durchgeführt, da in der Fachwelt die Meinung herrschte, daß ansonsten die Reaktionsgeschwindigkeit zu gering für eine wirtschaftlich befriedigende Anwendung des Verfahrens sei.

Bei diesen pH-Werten fallen regelmäßig hohe Mengen an Nebenprodukten an. Die Allylalkohole ihrerseits sind nämlich säureemfindlich und es werden bei der Verwendung starker Säuren Nebenprodukte gebildet, zum Beispiel durch intramolekulare Eliminierung von Wasser die entsprechenden Diene oder durch intermolekulare Eliminierung von Wasser oder Additionsreaktionen die entsprechenden Ether. Ebenso sind, sofern die Moleküle weitere C=C-Doppelbindungen enthalten, Gerüstumlagerungen möglich. M. Bertrand, B. Waegell und J. P. Zahra, Bull. Soc. Chim. Fr. 128 (1991) 904 - 910 beschreiben zum Beispiel die Umsetzung von DMVC in einer siedenden wäßrigen Lösung von 10 Gew.-% Oxalsäure zu Isopren (2-Methyl-1,3-butadien) und einem Gemisch verschiedener Alkohole der Terpenreihe. A.I Lebedeva und L.L. Shchukovskaya, l. c., stellten fest, daß das als Isomeres des DMVC gebildete Prenol bei pH-Werten von 1,27 und darunter in zunehmendem Maße zu Terpenen und Sesquiterpenen weiterreagiert.

Andere Verfahren versuchen, die Bildung von Nebenprodukten zu vermeiden. JP-A-54061110 lehrt ein Verfahren zur Isomerisierung von Allylalkoholen mit hohen Mengen an Borsäure als Katalysator. Verwendet werden 0,1 bis 60 Gew.-% Borsäure, insbesondere 1 bis 30 Gew.-%. Umsätze oberhalb von 90 % werden erst mit Borsäurekonzentrationen oberhalb von 7 Gew.-% Borsäure erreicht, bei noch höheren Borsäuremengen sinkt die Selektivität der Isomerisierung rapide.

Aus DE-A- 1 925 197 ist weiterhin ein Verfahren zur Herstellung von 2-Methyl-3-buten-2-ol (DMVC) durch Umsetzen von 3-Methyl-3-buten-1-ol (Isoprenol) in wäßriger Lösung in Gegenwart von einoder mehrbasischen Säuren bei Temperaturen von 20 bis 250 °C bekannt. Nachteilig an diesem Verfahren ist, daß sowohl die Umsätze als auch die Ausbeuten an DMVC unbefriedigend sind.

Auch durch Katalyse an Übergangsmetallverbindungen, zum Beispiel den in DE-A-25 16 698 offengelegten Wolframverbindungen, können nach den dort ebenfalls offengelegten Verfahren Allylalkohole isomerisiert werden. Nachteilig ist hier die aufwendige Synthese der Wolframkatalysatoren und der trotz der anzuwendenden hohen Temperaturen von über 150°C vergleichsweise niedrige Umsatz.

Es besteht daher nach wie vor ein großer Bedarf nach einem Verfahren, das die Isomerisierung von Allylalkoholen auf einfache, billige und möglichst selektive Weise bei dennoch hohen Raumzeitausbeuten ermöglicht, ohne daß hohe Katalysatormengen eingesetzt werden müssen. Insbesondere sollte dieses Verfahren die Herstellung von tertiären Allylalkoholen aus primären oder sekundären Allylalkoholen ermöglichen. Aufgabe dieser Erfindung war es, ein derartiges Verfahren zu finden.

Demgemäß wurde ein Verfahren zur Isomerisierung von Edukt-Allylalkoholen zu Produkt-Allylalkoholen in wäßriger Lösung in Gegenwart von Protonensäuren gefunden, das dadurch gekennzeichnet ist, daß man den pH-Wert des Reaktionsgemisches mit einer Protonensäure ausgewählt aus der Gruppe Flußsäure, Salzsäure, Perchlorsäure, Bromwasserstoffsäure, Schwefelsäure, Sulfonsäure, Salzen des Hydrogensulfations, Salpetersäure, Phosphorsäure, Salzen des Dihydrogenphosphats, Ameisensäure, Essigsäure, Propionsäure, Buttersäure, 2-Ethylhexansäure, Benzoesäure, Oxalsäure, Weinsäure, Bernsteinsäure, Maleinsäure, Fumarsäure, Adipinsäure oder Citronensäure in einer Konzentration unterhalb von 0,16 Mol pro Liter des Reaktionsgemisches auf einen Bereich von 2 bis 5 einstellt.

Das Verfahren zeigt überraschenderweise, daß zur Isomerisierung von Allylalkoholen mit Mineralsäuren als Katalysatoren die bisher angewandten niedrigen pH-Werte, die zu ausbeutevermindernden Nebenreaktionen führen, nicht nötig sind, und daß auch primäre Allylalkohole bei diesen pH-Werten auf zufriedenstellende Weise zu tertiären Allylalkoholen isomerisiert werden können. Die Anwendung des erfindungsgemäßen Verfahrens führt zu wirtschaftlich befriedigenden Raumzeitausbeuten und zu wirtschaftlich befriedigender Selektivität.

Das Verfahren zeigt weiterhin überraschenderweise, daß bei Verwendung von Puffern zum Einstellen des pH-Wertes auch bei hohen und/oder schwankenden Mengen an sauer oder basisch reagierenden Verunreinigungen im Edukt-Allylalkohol technisch aufwendige und/oder wirtschaftlich unbefriedigende Reinigungsmaßnahmen vermieden werden können oder ihre Notwendigkeit zumindest stark eingeschränkt werden kann.

Die im erfindungsgemäßen Verfahren einsetzbaren Edukt-Allylalkohole haben die allgemeine Formel I. Die Reste R¹, R², R³, R⁴ und R⁵ sind unabhängig voneinander Wasserstoff oder ein aliphatischer, cycloaliphatischer, aromatischer, araliphatischer oder heteroaromatischer Rest, zum Beispiel Alkyl, Alkenyl, Alkinyl, Cycloalkyl, Cycloalkenyl, Aryl wie zum Beispiel Phenyl, Alkaryl oder Aralkyl. Die Reste können mit weiteren organischen Resten substituiert sein und auch Heteroatome, zum Beispiel in Form von Alkoxisubstituenten. Estergruppen, Amino- oder Alkylaminofunktionen umfassen. Die Heteroatome wie Sauerstoff, Schwefel und Stickstoff können auch Teil eines aromatischen oder cyclischen Rests sein. Die Reste können untereinander auch verknüpft sein und Teile von einoder mehrgliedrigen, einfachen oder polycyclischen Ringsystemen, zum Beispiel mit 4 bis 16 Kohlenstoffatomen, welche die eingebundenen Reste und ein, zwei, oder drei C-Atome der Allyleinheit umfassen, sein.

Beispiele für im erfindungsgemäßen Verfahren verwendbare Edukt-Allylalkohole sind diejenigen, in denen die Reste R¹, R², R³, R⁴ und R⁵, jeweils unabhängig voneinander, Wasserstoff oder lineare gesättigte Alkylreste mit einem bis 18 Kohlenstoffatomen wie Methyl, Ethyl, Propyl, Butyl, Pentyl, Hexyl, Heptyl, Octyl, Nonyl oder Decyl sind. Die Reste können ebenso gesättigte cyclische Alkylreste mit drei bis 12 Kohlenstoffatomen wie beispielsweise Cyclopentyl, Cyclohexyl oder Cycloheptyl sein oder auch verzweigte gesättigte Alkylreste wie 2-Propyl, 2-Butyl, 2-Methyl-1-propyl, 1,1-Dimethylethyl oder alle verzweigten isomeren Pentyl-, Hexyl-, Heptyl-, Octyl-, Nonyl- oder Decylreste. Sie können auch ungesättigte Reste mit einer oder mehreren Doppel- und/oder Dreifachbindungen, die aus den oben genannten gesättigten Resten durch die formale Entfernung von mindestens zwei an benachbarten Kohlenstoffatomen befindlichen Wasserstoffatomen hervorgeht, sein, beispielsweise Vinyl, Propenyl, Butenyl, 1-Prop-2-enyl, 1-But-2-enyl oder 1-But-3-enyl. Sie können ebenso aromatische Reste sein, beispielsweise Phenyl oder 1- oder 2-Naphthyl. Die genannten Reste können jeweils unter den Reaktionsbedingungen inerte Substituenten tragen, zum Beispiel Halogen-, Alkyl- oder Alkoxisubstituenten.

Im erfindungsgemäßen Verfahren bevorzugt ist die Verwendung von primären Allylalkoholen der Formel I, bei denen R¹ und R² Wasserstoff sind, oder von sekundären Allylalkoholen der Formel I, bei denen entweder R¹ oder R² Wasserstoff und der andere Rest nicht Wasserstoff ist, wobei sowohl bei den primären als auch bei den sekundären Allylalkoholen R⁴ und R⁵ beide nicht Wasserstoff sind, als Edukt-Allylalkohole. In diesem Falle werden aus primären oder sekundären Allylalkoholen tertiäre Allylalkohole hergestellt.

Ein besonders bevorzugter Anwendungsfall für das erfindungsgemäße Verfahren ist die Synthese von DMVC aus Prenol. Ein weiterer bevorzugter Anwendungsfall ist die Synthese von Linalool aus Geraniol oder Nerol.

Als Katalysator können im erfindungsgemäßen Verfahren die nachfolgend aufgeführten Protonensäuren eingesetzt werden, mit denen sich das Reaktionsgemisch in der erfindungsgemäßen Menge in den erfindungsgemäßen pH-Bereich ansäuern läßt und deren Anionen inert gegenüber dem eingesetzten und dem produzierten Allylalkohol sind. Anstelle der reinen Säuren können auch Gemische der Säuren verwendet werden.

Beispiele für einzeln oder im Gemisch einsetzbare Protonensäuren sind Flußsäure, Salzsäure, Perchlorsäure, Bromwasserstoffsäure, Schwefelsäure, Sulfonsäuren, Salze des Hydrogensulfations wie zum Beispiel Kalium- oder Natriumhydrogensulfat, Salpetersäure, Phosphorsäure, Salze des Dihydrogenphosphats wie Kalium- und Natriumdihydrogenphosphat. Weitere Beispiele für verwendbare Säuren sind die organischen, von den Alkanen oder Aromaten abgeleiteten Säuren wie Ameisensäure, Essigsäure, Propionsäure, Buttersäure, 2-Ethylhexansäure, Benzoesäure, Oxalsäure, Weinsäure, Bernsteinsäure, Maleinsäure, Fumarsäure, Adipinsäure und Citronensäure.

Die Konzentration der verwendeten Säure liegt im allgemeinen unterhalb von 0,16 Mol pro Liter des Reaktionsgemisches, in bevorzugter Weise unterhalb von 0,12 Mol pro Liter und in besonders bevorzugter Weise unterhalb von 0,1 Mol pro Liter. Von starken Säuren, beispielsweise Schwefelsäure, sind zur Einstellung des erfindungsgemäßen pH-Bereichs sehr viel geringere Konzentrationen erforderlich. Von Schwefelsäure wird im allgemeinen eine Konzentration von etwa 0,01 Mol pro Liter oder weniger ausreichend sein. Schwächere Säuren, beispielsweise Dihydrogenphosphate, müssen in höheren Konzentrationen bis zu 0,16 Mol pro Liter verwendet werden, um den erfindungsgemäßen pH-Bereich einzustellen.

Die im erfindungsgemäßen Verfahren auch als Katalysatoren einsetzbaren Puffer sind solche Gemische aus Säure und Salzen ihrer konjugierten Basen oder Basen und Salzen ihrer konjugierten Säuren, die eine erfindungsgemäße Protonensäure oder protonensaure Funktionen enthalten und mit denen sich ein pH-Wert im erfindungsgemäß verwendeten pH-Bereich einstellen läßt. Im allgemeinen ist es für die Wirkung als Puffer erforderlich, daß mindestens ein Teil des konjugierten Säure-Base-Paars keine starke Säure oder Base ist. Der Begriff "stark" wird üblicherweise zur Kennzeichnung solcher Säuren gebraucht, die praktisch vollständig dissoziiert vorliegen, wobei bei einer mehrbasigen Säure auch nur eine Säurefunktion stark sein kann; entsprechend umgekehrt gilt der Begriff "stark" üblicherweise auch für entsprechende Basen. Diese Puffer können der Reaktionsmischung als Gemisch zugesetzt werden, genauso können die einzelnen Bestandteile des Puffers getrennt zugegeben werden oder in situ erzeugt werden, beispielsweise durch Zugabe einer starken Säure zum Salz einer schwachen Säure oder durch Zugabe einer starken Base zum Salz einer schwachen Base. Genauso können Puffer verwendet werden, die kein Gemisch darstellen, sondern saure und basische Funktionen vereinigen, beispielsweise Salze mehrbasiger Säuren, die noch saure Funktionen enthalten, oder Polymere, die sowohl saure als auch basische Funktionen enthalten. Eine ausführliche Definition des ansonsten allgemein bekannten Begriffs "Puffer" und eine Beschreibung der Wirkungsweise von Puffern ist Römpp's Chemie Lexikon, Band 5, Seite 3677, 9. Auflage, Stuttgart 1992, zu entnehmen.

Als Katalysator der Isomerisierung ist im erfindungsgemäßen Verfahren im Prinzip jeder Puffer geeignet, der einen Anteil an Protonensäure enthält, und mit dem im Reaktionsmedium ein pH-Wert im Bereich von 2 bis 5 eingestellt werden kann. Solche Puffer sind dem Fachmann bekannt und werden auch kommerziell vertrieben

Vorzugsweise werden Puffer verwendet, die oder deren Bestandteile mit Ausnahme der Katalyse der Isomerisierung inert gegenüber den eingesetzten oder produzierten Allylalkoholen sind. Die Inertheit kann gegebenenfalls durch Routineversuche geprüft werden.

Beispiele für erfindungsgemäß einsetzbare Puffer sind Gemische aus Phosphorsäure und Natriumphosphat ("Phosphatpuffer"), Milchsäure und Natriumlactat ("Lactatpuffer"), Citronensäure und Natriumcitrat ("Citratpuffer") oder Natriumacetat/Essigsäure ("Acetatpuffer") mit einem pH-Wert im Bereich von 2 bis 5.

Genauso können Salze mehrbasiger Säuren verwendet werden, in denen noch saure Funktionen vorhanden sind. Beispiele hierfür sind Alkalisalze mehrbasiger Carbonsäuren wie Mononatriumoxalat, Mononatriumtartrat, Mononatriumsuccinat, Mononatriumadipat, Mononatriumcitrat, Dinatriumcitrat, Mononatriumphthalat oder die entsprechenden Kaliumverbindungen, oder Salze anorganischer mehrbasiger Säuren wie Dinatriumhydrogenphosphat. Durch weitere Zugabe einer starken Säure, beispielsweise einer Mineralsäure wie Schwefelsäure oder Salzsäure, oder einer starken Base, beispielsweise Natron- oder Kalilauge, kann dabei der pH-Wert des Reaktionsmediums exakt auf den gewünschten Wert im Bereich von 2 bis 5 eingestellt werden. Beispiele für solche Puffer sind Dinatriumcitrat/Salzsäure, Kaliumhydrogenphthalat/Salzsäure oder Dinatriumhydrogenphosphat/Salzsäure.

Besonders bevorzugt ist die Verwendung eines Citratpuffers.

Die Menge des verwendeten Puffers ist im allgemeinen unkritisch. Sehr hohe Pufferkonzentrationen im Reaktionsmedium werden aus wirtschaftlichen Erwägungen meist unvorteilhaft sein, haben jedoch keinen technischen Nachteil, sofern die physikalischen Eigenschaften der Reaktionslösung (zum Beispiel Viskosität, Aboder Anwesenheit von ungelösten Feststoffen oder einer zweiten flüssigen Phase) nicht wesentlich beeinträchtigt werden. Im allgemeinen wird aber ein Gehalt des Reaktionsmediums an Puffer von maximal 30 Gew.-% ausreichend sein, in bevorzugter Weise werden maximal 20 Gew.-% Puffer und in besonders bevorzugter Weise maximal 10 Gew.-% Puffer verwendet.

Der im erfindungsgemäße Verfahren optimale einzustellende pH-Wert liegt im allgemeinen oberhalb von 2, insbesondere oberhalb von 2,0. Bevorzugterweise wird ein pH-Wert von mindestens 2,2 und in besonders bevorzugter Weise von mindestens 2,5 eingestellt. Der pH-Wert liegt im allgemeinen bei höchstens 5, in bevorzugter Weise bei höchstens 4,0 und in besonders bevorzugter Weise bei höchstens 3,5.

Die Wahl des pH-Werts hat Einfluß auf die Geschwindigkeit und die Selektivität der Reaktion. Ein niedriger pH-Wert von zum Beispiel 2,0 bedeutet hohe Reaktionsgeschwindigkeit, aber auch niedrigere Selektivität, insbesondere bei hohen Verweilzeiten des Produkts im Reaktor. Diese Vorgehensweise kann jedoch im Einzelfall auch vorteilhaft sein, insbesondere, wenn das Reaktionsgemisch rasch aufgearbeitet werden kann und das Produkt so schnell von der vorhandenen Säure getrennt wird. Wendet man zu hohe pH-Werte an, so erhält man das Produkt mit hoher Selektivität, aber niedrigen Raumzeitausbeuten. Im Einzelfall wird abzuwägen sein, welcher pH-Wert und insbesondere welche Kombination von pH-Wert, Temperatur und Verweildauer im Reaktor das Optimum von Raumzeitausbeute und Selektivität bewirkt.

Das erfindungsgemäße Verfahren kann diskontinuierlich oder kontinuierlich durchgeführt werden.

Man kann das Verfahren zum Beispiel so durchführen, daß man in einem Reaktionsgefäß die Säure bzw. den Puffer und das Lösungsmittel vorlegt und den zu isomerisierenden Allylalkohol zugibt. Es ist jedoch genauso möglich, den Allylalkohol und das Lösungsmittel vorzulegen und die Säure bzw. den Puffer zuzugeben oder das Lösungsmittel vorzulegen und den Allylalkohol und die Säure bzw. den Puffer gleichzeitig zuzugeben. Genauso können Gemische von Lösungsmittel und Allylalkohol oder von Lösungsmittel und Säure bzw. Puffer zugegeben werden. Im Einzelfall kann es, beispielsweise aufgrund entstehender und abzuführender Reaktionswärme, vorteilhaft sein, den Allylalkohol und/oder die Säure bzw. den Puffer oder ihre Gemische mit Lösungsmittel nicht auf einmal zuzugeben, sondern in mehreren Chargen oder kontinuierlich zu dosieren. Nach Beendigung der Reaktion wird das entstandene Produktgemisch aufgearbeitet.

Es kann je nach der gewählten Art der Aufarbeitung vorteilhaft sein, das Reaktionsmedium nach Beendigung der Reaktion neutral zu stellen, um säureinduzierte selektivitätsvermindernde Weiterreaktionen zu verhindern. Im allgemeinen genügt es dazu, den pH-Wert des Reaktionsmediums auf einen Wert größer 5 anzuheben. Dies kann durch Zugabe von alkalischen Verbindungen zum Reaktionsmedium erfolgen. Als alkalische Verbindungen sind zum Beispiel basisch reagierende Verbindungen wie Ammoniak, Natriumcarbonat, Natriumphosphat, Natriumhydroxid, die entsprechenden Kaliumverbindungen oder wäßrige Lösungen dieser Verbindungen geeignet.

Zur Aufarbeitung wird im allgemeinen das Produktgemisch vom Lösungsmittel der Reaktion abgetrennt. Dies kann zum Beispiel durch Destillation oder durch Extraktion mit einem Extraktionsmittel erfolgen. Als Extraktionsmittel sind prinzipiell alle Extraktionsmittel geeignet, in denen sich das Produktgemisch besser als in Wasser löst und die gegen das gewünschte Produkt, den Produkt-Allylalkohol, inert sind. Vorzugsweise werden Extraktionsmittel verwendet, von denen sich das gewünschte Produkt leicht, zum Beispiel durch Destillation oder Kristallisation, wieder abtrennen läßt. Im erfindungsgemäßen Verfahren besonders geeignete Extraktionsmittel sind organische Lösungsmittel, zum Beispiel offenkettige, verzweigte oder cyclische Dialkylether wie Diethylether, Dibutylether, tert.-Butylmethylether, tert.-Butylethylether, Tetrahydrofuran oder Dioxan, aliphatische, alicyclische oder aromatische Kohlenwasserstoffe wie Pentan, Hexan, Cyclopentan, Cyclohexan, Benzol, Toluol oder die Xylole, Ketone wie Aceton, Alkohole wie Butanol, oder andere Lösungsmittel wie Acetonitril, Dimethylsulfoxid, Dimethylformamid, N-Methylpyrrolidon oder Sulfolan. Das vom Produktgemisch befreite Lösungsmittel kann in die Reaktion zurückgeführt werden.

Im Anschluß an die Extraktion wird das Produktgemisch mit bekannten Verfahren, beispielsweise durch Destillation oder Kristallisation vom Extraktionsmittel abgetrennt.

Das vom Lösungsmittel der Reaktion befreite Produktgemisch kann zwar bei sehr hohen Umsätzen und Selektivitäten auch praktisch reiner Produkt-Allylalkohol sein, im allgemeinen werden aber Nebenprodukte und/oder unumgesetzter Edukt-Allylalkohol in kleineren Mengen enthalten sein. Daher wird sich im allgemeinen an die Isolierung des Produktgemisches aus dem Reaktionsmedium eine Reinigungsstufe, also eine Abtrennung des gewünschten Produkts anschließen. Diese Abtrennung und Reinigung des gewünschten Produkts kann nach bekannten Verfahren, zum Beispiel durch fraktionierende Destillation oder fraktionierende Kristallisation erfolgen.

Es kann im Einzelfall auch wirtschaftlich sinnvoll sein, im Produktgemisch in ausreichender Menge anfallenden unumgesetzten Edukt-Allylalkohol im selben Schritt wie das gewünschte Produkt oder in einem eigenen Abtrenn- und Reinigungsschritt in ausreichender Reinheit zu gewinnen und erneut in die Reaktion einzuführen.

Die Isomerisierung ist eine Gleichgewichtsreaktion. Die Lage des Gleichgewichts, also die sich einstellenden Konzentrationen von Edukt-Allylalkohol und Produkt-Allylalkohol, ist von deren thermodynamischen Eigenschaften und von den Reaktionsbedingungen abhängig. Durch diskontinuierliche oder vorzugsweise kontinuierliche Entfernung des Reaktionsprodukts aus dem Reaktionsmedium kann man die Isomerisierung auch in den Fällen, in denen die Lage des Gleichgewichts nur in unbefriedigendem Maße zur Bildung des gewünschten Produkts führt, dennoch mit zufriedenstellenden Raumzeitausbeuten zur Herstellung des gewünschten Produkts nutzen.

Eine kontinuierliche Durchführung des erfindungsgemäßen Verfahrens kann zum Beispiel so erfolgen, daß aus dem Reaktionsraum kontinuierlich ein Stoffstrom entnommen wird, der den oben beschriebenen Schritten der Abtrennung des Lösungsmittels und Auftrennung des Produktgemisches unterworfen wird. Auf diese Weise wird das gewünschte Produkt wie auch die eventuell anfallenden Nebenprodukte kontinuierlich aus dem Reaktionsraum entfernt. Unumgesetzter Edukt-Allylalkohol wird gemeinsam mit frischem Edukt-Allylalkohol und Lösungsmittel kontinuierlich in die Reaktion eingeführt. Der pH-Wert im Reaktionsgefäß kann zum Beispiel durch eine pH-Meßeinrichtung kontinuierlich überwacht und bei Abweichungen vom Sollwert, der im erfindungsgemäßen Bereich liegt, korrigiert werden. Diese Korrektur kann beispielsweise durch Zugabe von Puffer oder Bestandteilen des Puffergemisches erfolgen, wobei im letzteren Fall bei einem Absinken des pH-Werts ein basischer Pufferbestandteil und bei einem Ansteigen des pH-Werts ein saurer Pufferbestandteil zuzugeben sind.

Zur Vermeidung der Anreicherung unerwünschter Komponenten im Reaktionsmedium kann es vorteilhaft sein, einen Teil des Reaktionsmediums diskontinuierlich oder kontinuierlich aus dem Reaktionsraum zu entfernen und durch frisches oder nach Abtrennung der störenden Komponenten rezykliertes Reaktionsmedium zu ersetzen. Dies kann zum Beispiel durch Entsorgung oder Reinigung und Wiederaufarbeitung mindestens eines Teils des Stoffstroms erfolgen, der nach Abtrennung des Produktgemisches durch Destillation oder Extraktion verbleibt.

Eine besonders einfache Ausführungsform ergibt sich, wenn das gewünschte Produkt die Komponente des Reaktionsgemisches mit dem deutlich niedrigsten Siedepunkt ist. In diesem Fall kann das Verfahren einfach als kontinuierliche Destillation realisiert werden, wobei der Sumpf der Destillationskolonne als Reaktionsraum dient.

Die Isomerisierung wird im allgemeinen in wäßriger Lösung durchgeführt. Es kann zweckmäßig sein, zum Beispiel bei Verwendung von in Wasser schlecht löslichen Allylalkoholen, Mischungen von Wasser mit einem mit Wasser im verwendeten Mischungsverhältnis mischbaren organischen Lösungsmittel zu verwenden. Beispiele für verwendbare Lösungsmittel sind Acetonitril, Dimethylsulfoxid, Dimethylformamid, Tetrahydrofuran, N-Methylpyrrolidon, Ethylencarbonat, Propylencarbonat oder Sulfolan.

Die Konzentration des Allylalkohols im Lösungsmittel ist ein im Einzelfall zu optimierender Parameter. Bei zu hohen Konzentrationen wird vermehrt die Bildung von Nebenprodukten wie zum Beispiel die Kondensation zu Ethern, insbesondere bei Zweiphasigkeit des Gemisches, beobachtet. Bei zu niedrigen Konzentrationen fällt bei der Aufarbeitung ein unnötig großer, verdünnter Stoffstrom an, der größeren Aufwand zum Beispiel bei Zwischenlagerung, höheren Energieverbrauch, zum Beispiel beim Pumpen und höheren Aufwand bei der Aufarbeitung, zum Beispiel wegen der nötigen Extraktion aus einem verdünnteren Medium, bedeutet. Optimal scheint der Bereich an und unterhalb der maximalen Löslichkeit des eingesetzten Allylalkohols im verwendeten Lösungsmittel zu sein. Die verwendeten Konzentrationen werden letztendlich von wirtschaftlichen Überlegungen bestimmt, wobei im allgemeinen eine Obergrenze von 30 Gew.-% des Ausgangsstoffes im Reaktionsgemisch ausreichend sein wird, um eine zufriedenstellende Wirtschaftlichkeit zu erzielen.

Im allgemeinen kann die Reaktion bei Umgebungstemperatur durchgeführt werden. Zur Erzielung befriedigender Raumzeitausbeuten ist es jedoch im allgemeinen zweckmäßig, eine Temperatur oberhalb von 50°C anzuwenden. Bevorzugterweise wird die Temperatur oberhalb von 60°C gewählt. Im allgemeinen wird man eine Temperatur unterhalb des Siedepunkts des Reaktionsgemisches wählen, in der Regel unterhalb von 200°C, bevorzugt unterhalb von 100°C.

Der Druck ist im erfindungsgemäßen Verfahren kein kritischer Parameter, sondern kann zum Beispiel so gewählt werden, daß bei der angewendeten Reaktionstemperatur das Lösungsmittel siedet. Bevorzugterweise wird jedoch bei Normaldruck gearbeitet.

Das erfindungsgemäße Verfahren vermeidet die verschiedenen Nachteile der einzelnen bekannten Verfahren und ermöglicht es so, mit hoher Ausbeute und Selektivität Allylalkohole in wirtschaftlicher Weise zu isomerisieren.

### Beispiele

### Vergleichsbeispiel V1, Beispiele 1 bis 7: Einfluß des pH-Werts

In 500 ml Wasser wurde die zum Einstellen des gewünschten pH-Werts notwendige Menge Säure gelöst. Diese Mischung wurde in einem temperierbaren Reaktionsgefäß unter Rühren vorgelegt und auf 80°C erwärmt. Nach Erreichen der Temperaturkonstanz wurde der pH-Wert überprüft, in keinem Fall ergab sich eine Abweichung vom vorher eingestellten pH-Wert.

Zu dieser Mischung wurden 17,2 Gramm Prenol zugegeben. Die erhaltene Reaktionsmischung wurde weiter für 4 Stunden gerührt. Nach dieser Zeit wurden Proben gezogen, die sofort in einem Eisbad abgekühlt und anschließend sechsmal mit Diethylether extrahiert wurden. Die vereinigten organischen Extrakte wurden gaschromatographisch analysiert. Die Ausbeute- und Selektivitätsberechnungen, jeweils bezogen auf erhaltenes DMVC, beruhen auf den so ermittelten Flächenprozent-Werten der GC-Peaks der einzelnen Verbindungen.

Die Ergebnisse sind in der folgenden Tabelle I zusammengefaßt:

**Tabelle I**

| Bsp. | Säure | pH | Konzentration [Mol/l] | Ausbeute [%] | Selektivität [%] |
|---|---|---|---|---|---|
| V1*) | Phosphorsäure | 1,5 | 160,20·10⁻³ | 56 | 64 |
| 1 | Phosphorsäure | 2,5 | 40,00·10⁻³ | 74 | 91 |
| 2 | Phosphorsäure | 3,5 | 0,33·10⁻³ | 67 | 99 |
| 3 | Adipinsäure | 3,1 | 38,32·10⁻³ | 73 | 96 |
| 4 | Adipinsäure | 3,5 | 2,05·10⁻³ | 48 | 99 |
| 5 | Adipinsäure | 4,3 | 0,55·10⁻³ | 15 | 97 |
| 6 | Citronensäure | 3,5 | 0,83·10⁻³ | 73 | 98 |
| 7 | Salzsäure | 3,3 | 0,50·10⁻³ | 73 | 96 |

| | | | | | |
|---|---|---|---|---|---|
| *) Vergleichsbeispiel. Der Versuch wurde bereits nach 3 Stunden beendet. | | | | | |

Die Beispiele 1 bis 7 zeigen, daß praktisch unabhängig von der Art der eingesetzten Säure Ausbeute und Umsatz vom pH-Wert abhängig sind, und daß der optimale pH-Bereich von 2 bis 5 beträgt.

Das Vergleichsbeispiel V1 zeigt, daß bei niedrigem pH-Wert sowohl die Ausbeute wie auch und vor allem die Selektivität unvorteilhaft niedrig sind.

### Beispiele 8 bis 10: Einfluß der Temperatur

In 500 ml Wasser wurde die zum Einstellen eines pH-Werts von 3,5 notwendige Menge Phosphorsäure gelöst. Diese Mischung wurde in einem temperierbaren Reaktionsgefäß unter Rühren vorgelegt und auf die gewünschte Temperatur erwärmt.

Zu dieser Mischung wurden 17,2 Gramm Prenol zugegeben. Die Säurekonzentration betrug in allen Fällen 0,33 · 10-3 Mol pro Liter Reaktionsmischung. Die erhaltene Reaktionsmischung wurde weiter für 4 Stunden gerührt. Nach dieser Zeit wurden Proben gezogen, die sofort in einem Eisbad abgekühlt und anschließend sechsmal mit Diethylether extrahiert wurden. Die vereinigten organischen Extrakte wurden gaschromatographisch analysiert. Die Ausbeute- und Selektivitätsberechnungen, jeweils bezogen auf erhaltenes DMVC, beruhen auf den so ermittelten Flächenprozent-Werten der GC-Peaks der einzelnen Verbindungen.

Die Ergebnisse sind in der folgenden Tabelle II zusammengefaßt. Zur besseren Vergleichbarkeit ist Beispiel 2 mit aufgeführt.

**Tabelle II**

| Bsp. | Temperatur [°C] | pH | Ausbeute [%] | Selektivität [%]} |
|---|---|---|---|---|
| 2 | 80 | 3,5 | 67 | 99 |
| 8 | 70 | 3,5 | 36 | 99 |
| 9 | 60 | 3,5 | 16 | 99 |
| 10 | 50 | 3,5 | 5,8 | 99 |

Die Beispiele 2 und 8 bis 10 zeigen, daß man zur Erzielung eines befriedigenden Prenol-Umsatzes binnen 4 Stunden vorteilhafterweise eine Reaktionstemperatur oberhalb von 50 °C anwendet.

### Beispiele 11 und 12: Einfluß der Konzentration des Ausgangsstoffs

In 500 ml Wasser wurde die zum Einstellen eines pH-Werts von 2,5 notwendige Menge Phosphorsäure gelöst. Diese Mischung wurde in einem temperierbaren Reaktionsgefäß unter Rühren vorgelegt und auf 80 °C erwärmt.

Zu dieser Mischung wurden in einem Versuch (Beispiel 17) 34,4 Gramm Prenol und in einem anderen Versuch (Beispiel 18) 86 Gramm Prenol zugegeben. Die Säurekonzentration betrug in allen Fällen 40,00·10⁻³ Mol pro Liter Reaktionsmischung. Die Mischung des Beispiels 12 war zweiphasig. Die erhaltenen Reaktionsmischungen wurde weiter für 4 Stunden gerührt. Nach dieser Zeit wurden Proben gezogen, die sofort in einem Eisbad abgekühlt und anschließend sechsmal mit Diethylether extrahiert wurden. Die vereinigten organischen Extrakte wurden gaschromatographisch analysiert. Die Ausbeute- und Selektivitätsberechnungen, jeweils bezogen auf erhaltenes DMVC, beruhen auf den so ermittelten Flächenprozentwerten der GC-Peaks der einzelnen Verbindungen.

Die Ergebnisse werden in der folgenden Tabelle III mit dem Ergebnis von Beispiel 1 verglichen.

**Tabelle III**

| Bsp. | Prenolmenge [g] | pH | Ausbeute[%] | Selektivität[%] |
|---|---|---|---|---|
| 1 | 17,2 | 2,5 | 74 | 91 |
| 11 | 34,4 | 2,5 | 72 | 84 |
| 12 | 86 | 2,5 | 59 | 74 |

Die Beispiele 11 und 12 zeigen im Vergleich mit Beispiel 1, daß erhöhte Konzentrationen des Ausgangsstoffs und insbesondere solche Konzentrationen des Ausgangsstoffs, die zur Zweiphasigkeit der Reaktionsmischung führen, die Ausbeute und die Selektivität der Isomerisierung deutlich mindern.

## Patentansprüche

1. Verfahren zur Isomerisierung von Edukt-Allylalkoholen zu Produkt-Allylalkoholen in wäßriger Lösung in Gegenwart von Protonensäuren, **dadurch gekennzeichnet, daß** man den pH-Wert des Reaktionsgemisches mit einer Protonensäure ausgewählt aus der Gruppe Flußsäure, Salzsäure, Perchlorsäure, Bromwasserstoffsäure, Schwefelsäure, Sulfonsäure, Salzen des Hydrogensulfations, Salpetersäure, Phosphorsäure, Salzen des Dihydrogenphosphats, Ameisensäure, Essigsäure, Propionsäure, Buttersäure, 2-Ethylhexansäure, Benzoesäure, Oxalsäure, Weinsäure, Bernsteinsäure, Maleinsäure, Fumarsäure, Adipinsäure oder Citronensäure in einer Konzentration unterhalb von 0,16 Mol pro Liter des Reaktionsgemisches auf einen Bereich von 2 bis 5 einstellt.

2. Verfahren nach Anspruch 1, **dadurch gekennzeichnet, daß** man primäre oder sekundäre Edukt-Allylalkohole zu tertiären Produkt-Allylalkoholen isomerisiert.

3. Verfahren nach Anspruch 1, **dadurch gekennzeichnet, daß** man eine Reaktionstemperatur von mindestens 50°C anwendet.

4. Verfahren nach Anspruch 1, **dadurch gekennzeichnet, daß** man den Edukt-Allylalkohol im Reaktionsgemisch in einer Konzentration von bis zu 30 Gew.-% vorliegen hat.

5. Verfahren nach Anspruch 1, **dadurch gekennzeichnet, daß** man als Edukt-Allylalkohol 3-Methyl-but-2-en-1-ol verwendet und als Produkt-Allylalkohol 2-Methyl-but-3-en-2-ol herstellt.

6. Verfahren nach Anspruch 1, **dadurch gekennzeichnet, daß** man als Säure Phosphorsäure verwendet.

7. Verfahren nach Anspruch 1, **dadurch gekennzeichnet, daß** man als Puffer einen Citratpuffer verwendet.

## Claims

1. A process for the isomerization of starting allyl alcohols to give product allyl alcohols in aqueous solution in the presence of protic acids, which comprises adjusting the pH of the reaction mixture to a range from 2 to 5 using a protic acid chosen from the group consisting of hydrofluoric acid, hydrochloric acid, perchloric acid, hydrobromic acid, sulfuric acid, sulfonic acid, salts of the hydrogensulfate ion, nitric acid, phosphoric acid, salts of dihydrogenphosphate, formic acid, acetic acid, propionic acid, butyric acid, 2-ethylhexanoic acid, benzoic acid, oxalic acid, tartaric acid, succinic acid, maleic acid, fumaric acid, adipic acid or citric acid in a concentration of less than 0.16 mol per liter of the reaction mixture.

2. A process as claimed in claim 1, wherein primary or secondary starting allyl alcohols are isomerized to give tertiary product allyl alcohols.

3. A process as claimed in claim 1, wherein a reaction temperature of at least 50°C is used.

4. A process as claimed in claim 1, wherein the starting allyl alcohol is present in the reaction mixture in a concentration of up to 30% by weight.

5. A process as claimed in claim 1, wherein the starting allyl alcohol used is 3-methylbut-2-en-1-ol, and the product allyl alcohol prepared is 2-methylbut-3-en-2-ol.

6. A process as claimed in claim 1, wherein the acid used is phosphoric acid.

7. The process as claimed in claim 1, wherein the buffer used is a citrate buffer.

## Revendications

1. Procédé pour isomériser des alcools allyliques, produits de départ, en alcools allyliques, produits de réaction, en solution aqueuse en présence d'acides protoniques, **caractérisé par le fait que** l'on règle le pH du mélange de réaction dans l'intervalle de 2 à 5 à l'aide d'un acide protonique choisi dans le groupe consistant en l'acide fluorhydrique, l'acide chlorhydrique, l'acide perchlorique, l'acide bromhydrique, l'acide sulfurique, un acide sulfonique, un sel de l'ion hydrogénosulfate, l'acide nitrique, l'acide phosphorique, un sel de dihydrogénophosphate, l'acide formique, l'acide acétique, l'acide propionique, l'acide butyrique, l'acide 2-méthylhexanoïque, l'acide benzoïque, l'acide oxalique, l'acide tartrique, l'acide succinique, l'acide maléique, l'acide fumarique, l'acide adipique ou l'acide citrique, à une concentration inférieure à 0,16 mol par litre du mélange de réaction.

2. Procédé selon la revendication 1, **caractérisé par le fait que** l'on isomérise des alcools allyliques primaires ou secondaires , produits de départ, en alcools allyliques tertiaires, produits de réaction.

3. Procédé selon la revendication 1, **caractérisé par le fait que** l'on opère à une température de réaction d'au moins 50° C.

4. Procédé selon la revendication 1, **caractérisé par le fait que** l'alcool allylique, produit de départ, est présent dans le mélange de réaction à une concentration allant jusqu'à 30 % en poids.

5. Procédé selon la revendication 1, **caractérisé par le fait que** l'alcool allylique de départ est le 3-méthyl-but-2-én-1-ol et l'alcool allylique produit le 2-méthyl-but-3-én-2-ol.

6. Procédé selon la revendication 1, **caractérisé par le fait que** l'acide utilisé est l'acide phosphorique.

7. Procédé selon la revendication 1, **caractérisé par le fait que** l'on utilise en tant que tampon un tampon citrate.
